# EUROPEAN PATENT SPECIFICATION

(11) **EP 3 934 728 B1**
(45) Date of publication and mention of the grant of the patent: **22.05.2024**
(21) Application number: 20766367.5
(22) Date of filing: 13.02.2020
(51) Int. Cl.: A61M 25/10, A61M 1/36, A61M 25/00, A61M 25/04

(54) **FEMORAL AND VENOUS ARTERIAL CANNULAS FOR MITIGATING THE RISK OF LIMB ISCHEMIA**
FEMORALE UND VENÖSE ARTERIELLE KANÜLEN ZUR VERMINDERUNG DES RISIKOS AUF GLIEDMASSENISCHÄMIE
CANULES ARTÉRIELLES FÉMORALES ET VEINEUSES VISANT L'ATTÉNUATION DU RISQUE D'ISCHÉMIE DES MEMBRES

(30) Priority: 05.03.2019 CA 3035749
(43) Date of publication of application: 12.01.2022
(73) Proprietor: Total Flow Medical Limited, Vancouver, British Columbia V6C 0A3 (CA)
(72) Inventor: HARRIS, Luke, Vancouver, British Columbia V5T 0G5 (CA); CURTIS, Andy, Vancouver, British Columbia V5T 0G5 (CA)
(74) Representative: Boult Wade Tennant LLP
(86) International application number: PCT/CA2020/050191
(87) International publication number: WO 2020/176968

(56) References cited:
- WO-A1-2016/022797
- WO-A1-2018/169503
- CN-U- 203 447 625
- DE-A1-102016 103 560
- US-A- 5 584 803
- US-A- 5 928 181
- US-A1- 2002 016 566
- MAGOVERN ET AL: "A femoral artery cannula that allows distal blood flow", THE JOURNAL OF THORACIC AND CARDIOVASCULAR SURGERY, MOSBY-YEAR BOOK, INC., ST. LOUIS, MO, US, vol. 130, no. 3, 1 September 2005 (2005-09-01), pages 684-686, XP005087659, ISSN: 0022-5223, DOI: 10.1016/J.JTCVS.2005.03.022

## Description

### COPYRIGHT NOTICE

This patent document contains material which is subject to copyright protection. The copyright owner has no objection to the facsimile reproduction by anyone of this patent document as it appears in the Patent and Trademark Office patent file or records, but otherwise reserves all copyright rights whatsoever.

### FIELD OF THE INVENTION

The invention relates to the field of cannulas and more specifically to femoral and venous arterial cannulas and a method for mitigating the risk of limb ischemia. The invention provides femoral arterial and venous cannulas for cardiopulmonary support. Both cannulas utilize a balloon to isolate femoral artery and vein circulations from their respective systemic arterial and venous flow; subsequently these cannulas provide for dedicated femoral artery perfusion along with dedicated femoral vein drainage. The cannulas avoid the need to insert a distal limb perfusion cannula to facilitate arterial limb perfusion and therefore mitigate the complications associated therewith.

### BACKGROUND OF THE INVENTION

The use of extracorporeal membrane oxygenation (ECMO) in both acute and chronic based cardiac and respiratory failure is a world wide established and proven life saving measure. As reported by the Extracorporeal Life Support Organization, both the number of registered ECMO centers as well as the number of ECMO runs has dramatically increased since 1990. Consequently, the expected frequency of complications associated with such treatment has concomitantly and predictably increased. (www.ELSO.org)

ECMO is often fraught with a range of complications ranging in severity from bleeding to sepsis to death (Juo, *et al.* 2017). A major contributor to ECMO related morbidity and mortality is the development of the ischemic limb. The presence of a femoral arterial and venous cannula to achieve adequate cardiac output can significantly impair blood flow and venous drainage from the leg. This presents numerous challenges to the clinician. If not identified early, the consequences of limb ischemia are numerous, often requiring surgical intervention and challenges with blood pressure and hemodynamic patient management.

To facilitate access to the vascular system for various types of cardiopulmonary support it is necessary to gain access to both the arterial and venous system of a patient. It is a common procedure to position cannula in arteries and veins. These cannulas are designed to drain from and deliver blood to the circulation. There are alternative techniques beyond the use of a single arterial cannula, e.g. arterial graft anastomosed in a sideway manner to the femoral artery, dual arterial cannula, however the clinical status of the patient will often preclude the additional time required to facilitate such practice. In addition to this each and every additional intervention is associated with additional morbidities. There are on occasions clinical reasons to use more than one venous cannula however this is a practice more commonly associated with specific open-heart surgery procedures.

Positioned between these cannulas are various devices that facilitate and control blood flow (a mechanical pump) as well as gas and temperature exchange (mechanical oxygenator with an incorporated heat exchanger). There may or may not be various types of reservoirs and filters required, this is dependent upon the type of support necessary for the patient's clinical status. These devices are connected to form a cardiopulmonary circuit with various lengths of tubing to join these multiple components.

Depending upon the reason for initiating cardiopulmonary support it is often necessary to introduce these cannulas into the femoral artery and femoral vein. This can be to expedite the institution of the necessary cardiopulmonary support (ECMO - Extracorporeal Membrane Oxygenation) or may be for an elective procedure due to the nature of the cardiac surgery that is to be performed, i.e. redo OHS (open heart surgery), MICS (minimally invasive cardiac surgery), porcelain aorta, etc.

For multiple and complex clinical reasons the drainage from and delivery of blood to the ipsilateral limb into which these cannulas have been placed can be seen to be clinically inferior to the patient's needs, i.e. inadequate arterial flow into the limb along with inadequate venous drainage from the limb.

The cause of these events is often mechanical in nature; the presence of the cannula within the lumen of the blood vessel can obliterate the blood pathway thus inhibiting the ability to either deliver blood downstream (retrograde) from the point of cannula insertion in the artery or to drain blood from the vein distal to the point of cannula entry.

Emergent cannulation of these vessels can often be traumatic resulting in damage to the vessel integrity along with vascular spasm. Limited evidence exists to suggest Body type and BMI are recognized as precipitators of limb mal-perfusion. The body of evidence strongly implies, however, that the ability to predict those susceptible to limb ischemia is highly ambiguous. This evidence highlights the need to institute both arterial and venous cannula designs shown below in all patients requiring ECMO or CPB. The use of dual arterial cannula (distal perfusion cannula) is complex and requires additional interventions; this is often precluded by the compromised nature of the patient's cardiac status.

The incidence of limb ischemia is a common morbidity in the presence of femoral cannulation and is multi-factorial in nature. It is recognized that the diminished blood flow to the limb is significant in nature but is not the sole factor in precipitating limb ischemia. This feature when combined with reduced or negligible venous drainage from the limb renders it highly susceptible to the consequence of mal-perfusion.

Compromised venous drainage of the limb results in limb engorgement with an overall increase in vascular pressure. This increase subsequently impacts upon the ability to deliver arterial flow while also inhibiting lymphatic drainage.

The consequence of limb ischemia is significant. Venous thrombosis is a common morbidity, while tissue necrosis would be the ultimate sequelae. Further interventions are routinely required in the presence of limb ischemia such as embolectomies, fasciotomies and amputations.

Accordingly, a need exists for a novel cannulation design and a method of using the same to ensure adequate arterial flow and venous drainage is maintained to the limbs during peripherally cannulated ECMO, open heart surgery (OHS) and minimally invasive heart surgery (MICS). Other objects of the invention will be apparent from the description that follows.

WO 2018/169503 describes a femoral arterial ECMO cannula with a balloon that has dual flow and that provides arterial body blood flow during femoral Extra Corporeal Membrane Oxygenation.

*"*A femoral artery cannula that allows distal blood flow" by Magovern et al., THE JOURNAL OF THORACIC AND CARDIOVASCULAR SURGERY, vol. 130, no. 3, 1 September 2005, pages 684 - 686, describes that a femoral artery cannula is used for certain types of circulatory support but can cause ischemia, especially during prolonged perfusion. The study tests the function of a femoral cannula designed to allow proximal and distal blood flow.

DE 10 2016 103560 describes a system for use in organ replacement or support procedures. The system comprises an extracorporeal organ function replacement or support device and a withdrawal catheter which has at least two catheter lumens surrounded by a catheter wall and a withdrawal opening having a first section for insertion into the vessel of the patient, the first section having at least one side opening in addition to the removal opening. The catheter wall, via which blood can be drained from the vessel into a first lumen. Furthermore, a first expandable element is provided in section, which can be expanded by supplying a fluid via a second lumen.

WO 2016/022797 describes multi-lumen cannulae that can be used in various different medical procedures. The multi-lumen cannulae can comprise an elongated body comprising multiple different ports that connect to the various different sidewall lumens contained within the elongated body. The multi-lumen cannulae can also comprise a central lumen that extends through the entire elongated body and can be fluidly connected to the various different sidewall lumens. The multi-lumen cannulae can further comprise two balloons on the exterior of the elongated body, which can be used to isolate the right atrium of a patient's heart.

### SUMMARY OF THE INVENTION

According to the present invention there is provided a femoral arterial cannula. The femoral arterial cannula may include an elongated hollow cylindrical cannula body having distal and proximal open ends. A low-profile balloon may be situated on the body adjacent the distal end and an inflation device may be connected to the balloon for inflating the balloon. Additionally, four blood flow exit ports may be situated in the cannula body between the balloon and the distal end with two of the distal exit ports in-line with one-another along the cannula body and two other distal exit ports being 180° circumferentially offset from the first two distal exit ports. Two blood flow exit ports may be situated in the cannula body between the balloon and the proximal end with the proximal exit ports being 180° circumferentially offset and longitudinally offset from one-another.

The cannula body may include a taper from the distal to the proximal end. The cannula body may also include a distal single radio-opaque marker and a proximal double radio-opaque marker.

The inflation device may include a small-bore tubing connected to the balloon at a first end, a balloon cuff connected to a second end of said small bore tubing, a two-way valve connected to the balloon cuff, and a female luer port connected to the two-way valve.

In accordance with another embodiment of invention there is provided femoral venous cannula. The femoral venous cannula may include an elongated hollow cylindrical cannula body of varying lengths having distal and proximal open ends. A broad-profile balloon may be situated on the body adjacent the proximal end and an inflation device may be connected to the balloon for inflating said balloon. Additionally, two blood flow entry ports may be situated in the cannula body between the balloon and the distal end with the distal entry ports being 180° circumferentially offset and longitudinally offset from one-another along the cannula body. Two blood flow entry ports may be situated in the cannula body between the balloon and the proximal end with the proximal entry ports being 180° circumferentially offset and longitudinally offset from one-another and 90° offset from the distal entry ports.

The cannula body may include a taper from said distal to said proximal end. The cannula body may also include a distal single radio-opaque marker and a proximal double radio-opaque marker.

The inflation device may include a small-bore tubing connected to said balloon at a first end, a balloon cuff connected to a second end of the small bore tubing, a two-way valve connected to the balloon cuff, and a female luer port connected to the two-way valve.

A method for mitigating the risk of limb ischemia is also described, said method not forming part of the claimed invention. The method may include inserting a femoral arterial cannula into a femoral artery of a limb with the femoral arterial cannula having a balloon to seal the femoral artery. Once in situ in the femoral artery, the method may include inflating the femoral arterial cannula balloon to seal the femoral artery to deliver blood flow in a retrograde direction to the limb while simultaneously delivering systemic arterial blood flow. Additionally, the method may include inserting a femoral venous cannula into a femoral vein of the limb with the femoral venous cannula having a balloon to seal the femoral vein. Once in situ in the femoral vein, the method may include inflating the femoral venous cannula balloon to seal the femoral vein to drain venous blood flow from the limb while simultaneously draining systemic venous blood flow.

Other aspects of the invention will be appreciated by reference to the detailed description of the preferred embodiment and to the claims that follow.

### BRIEF DESCRIPTION OF THE DRAWINGS

The preferred embodiment of the invention will be described by reference to the drawings thereof in which:
Fig. 1 is a femoral arterial cannula of the present invention;
Fig. 2 is a cross-sectional view along line 2-2 of Fig. 1;
Fig. 3 is a perspective view of the femoral arterial cannula of Fig. 1 with an inflated low-profile balloon;
Fig. 4 is the femoral arterial cannula of Fig. 1inserted and in situ in a femoral artery;
Fig. 5 is an isolation of the tip of the femoral arterial cannula of Fig, 1 rotated 90 degrees;
Fig. 6 is a femoral venous cannula of the present invention;
Fig. 7 is a cross-sectional view along line 7-7 of Fig. 6;
Fig. 8 is a perspective view of the femoral venous cannula of Fig. 6 with an inflated broad-profile balloon;
Fig. 9 is the femoral venous cannula of Fig. 6 inserted and in situ in a femoral vein;
Fig. 10 is another embodiment the femoral venous cannula of the present invention; and
Fig. 11 is a cross-sectional view along line 10-10 of Fig. 10.

### DESCRIPTION OF THE PREFERRED EMBODIMENT OF THE INVENTION

Ischemia is defined as, "an inadequate blood supply to an organ or part of the body". Tissue injury and/or death occur as a result of the initial ischemic insult.

The extent of injury is determined primarily by the magnitude and duration of the interruption in the blood supply. If unaddressed, cell death by necrotic, necroptotic, apoptotic, and autophagic mechanisms occurs (Kalogeris, *et al.* 2017).

Identification of limb ischemia caused by the presence of a femoral arterial cannula is of upmost importance with a universal recognition of the severity of consequences if not addressed early on. Given that this demographic of patients are most likely under general anesthesia the early signs of ischemia such as pain and paresthesia are often not possible to discern. This therefore relies on due diligence and frequent inspection of the patient's limbs. Regular limb inspection is essential, assessing for pallor, reduced temperature, slow capillary refill and assessment of pulses via palpation or doppler.

More advanced stages of ischemia include mottled skin, increased limb circumference and compartment pressures above 30mmHg. Accumulation of inflammatory mediators in a hypoxic environment in conjunction with the inability to wash out these mediators via the congested lymphatic system results in both apoptotic and necrotic cell death. This can cause an increase in systemic potassium. Both inflammatory mediators and high potassium can result in significant vasoplegia similar to that of sepsis. This often requires high dose vasopressors to maintain adequate systemic perfusion pressures. If distal limb flow is re-established late on in the ischemic insult the subsequent wash out of inflammatory mediators and potassium accumulation can further potentiate the vasoplegia (Kalogeris, *et al.* 2017).

Dependent on the extent at which the femoral artery and veins are occluded will determine the rate at which clinical signs for ischemia present themselves (Haley, *et al.*). This may be why ability to identify limb ischemia early on is fraught with difficulties. For example, a limb may present with reduced temperature and slow capillary refill, but integrity of pulses/flow are present via doppler and compartment pressures may be low. This may prompt a clinician to delay intervention given the potential for complications associated with the required intervention. The point at which intervention occurs therefore can vary significantly. Given the difficulty to reliably assess limb ischemia and varying degrees at which identifiable signs present themselves explains in part why some institutions consistently place a distal limb catheter as a prophylactic measure to ensure adequate limb flow is maintained.

### (ECMO) and Limb Ischemia Reporting and Incidence

The incidence of reported limb ischemia in ECMO patients seems to vary significantly across ECMO centres.

The North American 2017 ELSO report on Adult V-A ECMO complications shows a 5.5% incidence of limb ischemia with a 21% survival rate.

This incidence seems low compared to publications on limb ischemia. A review of reported limb ischemia incidence by Lamb *et al*.2016 et al. reported a range of limb ischemia events from 10-70% of patients that receive peripherally cannulated ECMO. In a meta analysis of limb ischemia incidence (Juo et a1.2017) reported limb ischemia in 17% of patients out of a total 1886 patients. Possible reasons for discrepancies in reporting maybe due to compounding complications and primary causes of mortality rates in ECMO patients. The potential for limb ischemia being unidentified or shadowed by other causes of mortality may result in under reporting. Furthermore, there may also be significant differences in cannulation technique, size of cannula, point at which the cannula is inserted, native anatomy nuances as well as the timing with which distal limb catheters are inserted.

It is however noted that the presence of limb ischemia in the absence of other ECMO related complications is devastating and considered an independent risk factor for mortality (Juo *et al.*)

| **Table 1.** Patient Demographics | | | | | |
|---|---|---|---|---|---|
| Variable | | Total (N= 151) | Rescue strategy (N = 107) | Preemptive strategy (N=44) | P |
| Age (years) | | 54.6 ± 16.3 | 57.2 ± 15.1 | 48.1 ± 17.4 | 0.003 |
| Male | | 90 (59.6) | 64 (59.8) | 26 (59.1) | 0.935 |
| Height (m) | | 1.6 ± 0.1 | 1.6 ± 0.1 | 1.6 ± 0.1 | 0.985 |
| BSA | | 1.7 ± 0.2 | 1.7 ± 0.2 | 1.6 ± 0.1 | 0.291 |
| BMI | | 23.2 ± 4.0 | 23.6 ± 4.1 | 22.4 ± 3.6 | 0.102 |
| APACHE II | | 13.0 ± 6.3 | 12.2 ± 6.0 | 14.8 ± 6.9 | 0.026 |
| SOFA | | 11.6 t 3.5 | 11.7 ± 3.5 | 11.3 ± 3.3 | 0.585 |
| Ischemia risk factor | | | | | |
| | Diabetes | 25 (16.4) | 21 (19.6) | 4 (9.1) | 0.113 |
| | Hypertension | 39 (25.7) | 32 (29.9) | 7 (15.9) | 0.074 |
| | Chronic renal insufficiency | 6 (3.9) | 5 (4.7) | 1 (2.3) | 0.493 |
| | Smoker | 27 (17.8) | 20 (18.7) | 7 (15.9) | 0.685 |
| | PVD | 11 (7.2) | 9 (8.4) | 2 (4.5) | 0.406 |
| | Cerebral infarction | 5 (3.4) | 5 (4.8) | 0 | 0.139 |
| Catheter size (Fr.) | | 17.4 ± 2.0 | 17.2 ± 2.1 | 17.9 ± 1.8 | 0.059 |
| Diameter of catheter (mm) | | 5.8 ± 0.7 | 5.7 ± 0.7 | 6.0 ± 0.6 | 0.052 |
| Cannula size (Fr.)-to-BSA ratio | | 10.6 ± 1.5 | 10.4 ± 1.6 | 11.0 ± 1.2 | 0.056 |
| Diameter of cannula (mm)-to-BSA ratio | | 3.5 ± 0.5 | 3.5 ± 0.5 | 3.7 ± 0.4 | 0.050 |
| ECMO days | | 5.2 ± 5.0 | 4.9 ± 4.9 | 6.0 ± 5.4 | 0.212 |
| Vasopressor use | | 44 (29.1) | 29 (27.1) | 15 (34.1) | 0.390 |
| Dialysis | | 46 (30.3) | 33 (30.8) | 13 (29.5) | 0.875 |
| Aspirin use | | 29 (19.1) | 19 (17.8) | 10 (22.7) | 0.481 |
| Dual anti-platelet drug use | | 23 (15.1) | 17 (15.9) | 6 (13.6) | 0.726 |
| Warfarin use | | 2 (1.3) | 1 (0.9) | 1 (2.3) | 0.513 |
| Successful weaning | | 98 (64.9) | 61 (57.0) | 37 (84.1) | 0.002 |
| Overall mortality | | 83 (55.0) | 66 (61.7) | 17 (38.6) | 0.010 |
| BSA. body surface area; BMI. body mass index; PVD, peripheral vascular disease; APACHE II, Acute Physiology and Chronic Health Evaluation II score; SOFA. Sequential Organ Failure Assessment score: ECMO, extracorporeal membrane oxygenation. Data are presented as numbers (with percentages) or as means ± standard deviation. | | | | | |

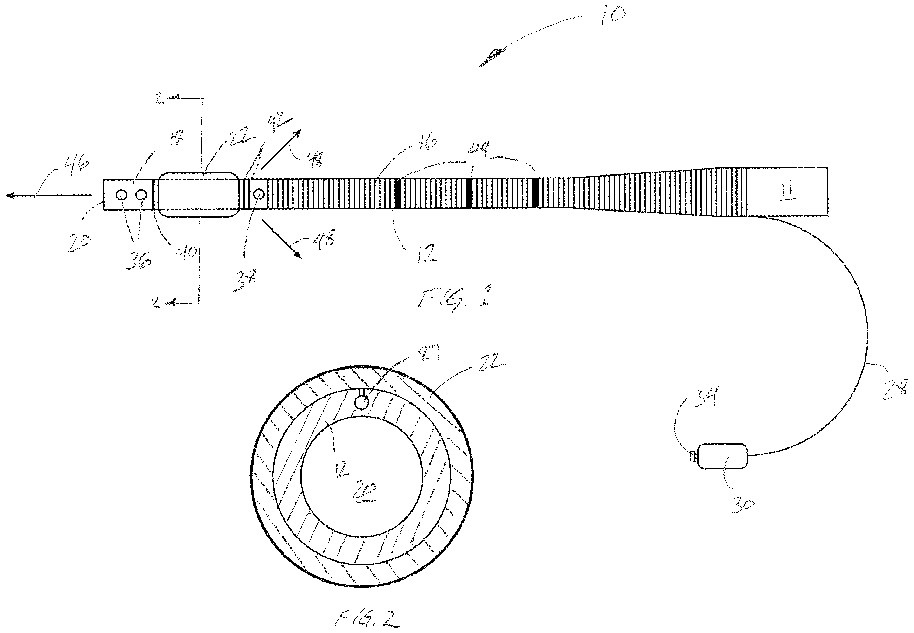

Figure 1: Patient demographics taken from Yuo *et al*.2016. This table demonstrates that expected co-morbidities that are expected to increase likelihood of limb ischemia are non-correlating.

### Risk Factors for Limb Ischemia

The identification of risk factors for the generation of limb ischemia is highly ambiguous. Presumed risk factors such as presence of obesity, diabetes, peripheral vascular disease and age does not seem to correlate with a higher incidence of limb ischemia. This is noted by Gander *et al* (2010) who experienced 52% incidence of limb ischemia and noted that, "No variable was predictive of the development of significant limb ischemia". It could be stipulated that the inherent risk could be solely due to the diameter of the femoral artery in relation to the size of the femoral arterial cannula as well as the point of insertion.

Conversely, the development of limb ischemia could be more complex than solely reduced arterial flow to the limb. This is noted by the prevalence of reported limb ischemia in Veno-Venous ECMO via ELSO.org. This suggests that the generation of limb ischemia could also be caused by venous and lymphatic congestion due to the presence of the femoral venous cannula.

Arguably it appears that there are a myriad of factors that result in limb ischemia which will likely explain why there are varying degrees of reported ischemic insults and degrees of success when surgical intervention and institution of a distal limb perfusion catheter is inserted.

This is supported by Yuo et al.2016 who examined the difference between ECMO patients that received immediate placement of a distal limb perfusion catheter and those who had one inserted as a rescue strategy at the first signs of limb ischemia.

The table (Figure 2) from Yuo et al.2016 demonstrates that a rescue strategy was not as successful as a pre-emptive strategy. The patients that received a distal limb catheter at the time of ECMO initiation had zero incidence of limb ischemia and therefore required no further surgical intervention such as fasciotomy or amputation. It is also notable that the difference in mortality was significantly lower in the preemptive strategy.

| **Table 2.** The Cannula-Related Complications Developing During ECMO Support | | | | | |
|---|---|---|---|---|---|
| | | Total (N - 151) | Rescue strategy (N - 107) | Preemptive strategy (N - 44) | P |
| Ischemia | | 10 (6.6) | 10 (9.3) | 0 | 0.036 |
| Surgical intervention | | 3 (2.0) | 3 (2.8) | 0 | 0.262 |
| | Fascioloniy | 2 (1.3) | 2 (1.9) | 0 | 0.359 |
| | Amputation | 1 (0.7) | 1 (0.9) | 0 | 0.248 |
| Cannulation site bleeding | | 14 (9.3) | 9 (8.4) | 5 (11.4) | 0.570 |
| Data are presented as numbers (with percentages). | | | | | |

Figure 2: Yuo *et al*.2016 demonstrating the difference between a rescue and preemptive strategy to address ischemic limb as a consequence to peripherally cannulated ECMO.

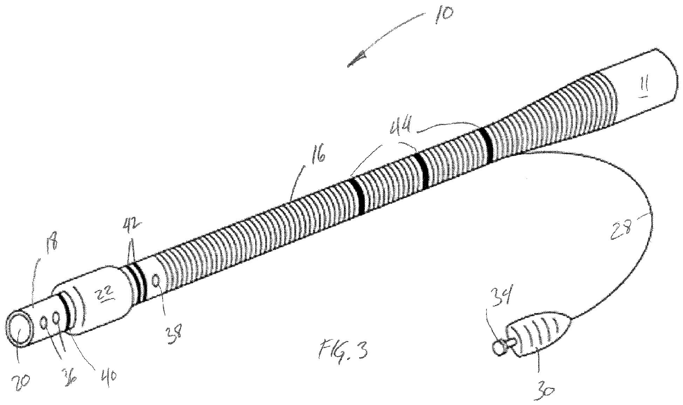

This finding was also found by Lamb et al.2017 who through examination of their ECMO patients demonstrated that immediate institution of a distal limb cannula prevents the development of limb ischemia in 100% of their patient's versus those who did not receive a distal limb cannula where 50% developed ischemic limb complications that required intervention including fasciotomies. (See Figure 3)

### Distal Limb Insertion Techniques and Complications

The insertion of a distal limb perfusion cannula is not without complication. Lamb *et al.* 2017 note that femoral vessel complications associated with cannulation include arterial dissection, pseudo-aneurysms, thromboembolic complications and infectious arterial complications. It could be surmised that these complications are inclusive of placement of a distal limb cannula.

Furthermore the manner with which flow to the limb is incorporated into the ECMO circuit can expose the patient to potential hazards and requires due care 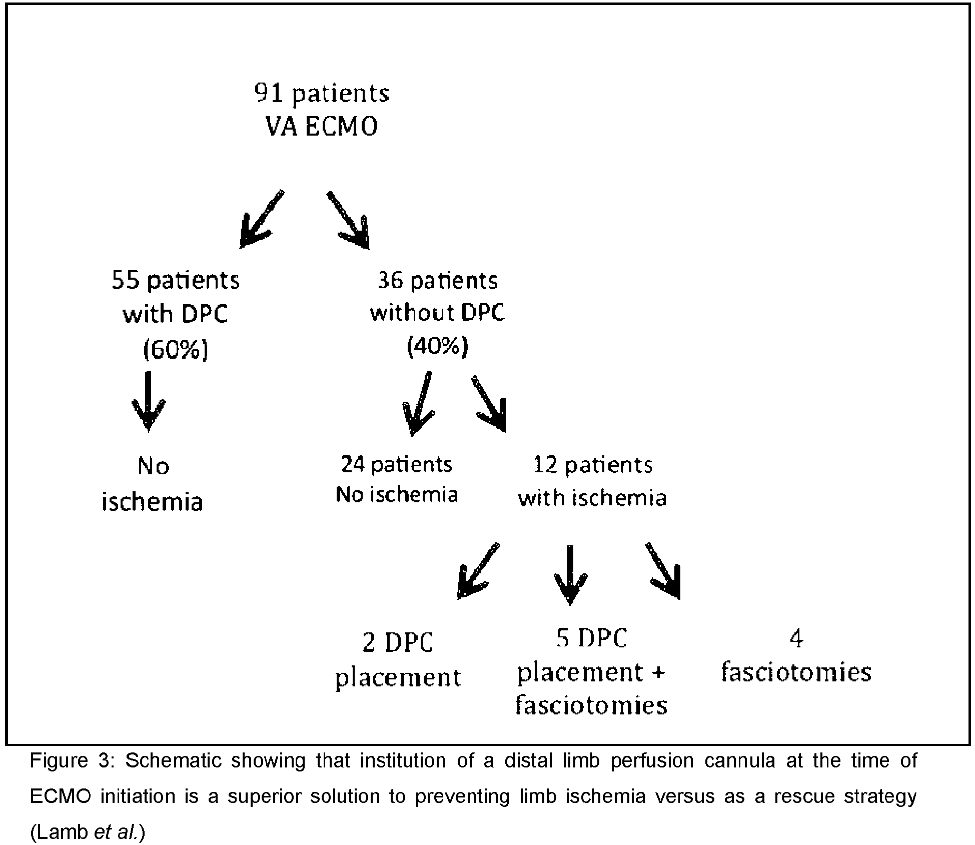 and attention by the Perfusionist to ensure the integrity of the ECMO circuit is maintained.

Given that the limb perfusion cannula provides flow via extra 1/4 inch tubing either via a Y connector off the main 3/8 inch tubing or an external port on the ECMO oxygenator, if the flow is not sufficient it can often result in clot formation and potentially embolic delivery to the limb. This can often compound limb ischemia and requires complex intervention by a vascular surgeon to perform embolectomies and or femoral bypass grafts. This can further expose the patient to excessive bleeding, potential for infection as well as increased blood transfusion rates. It is not uncommon for ECMO patients to receive multiple allogeneic blood transfusions. Given this patient demographic can be often worked up for heart transplant candidacy, increased blood 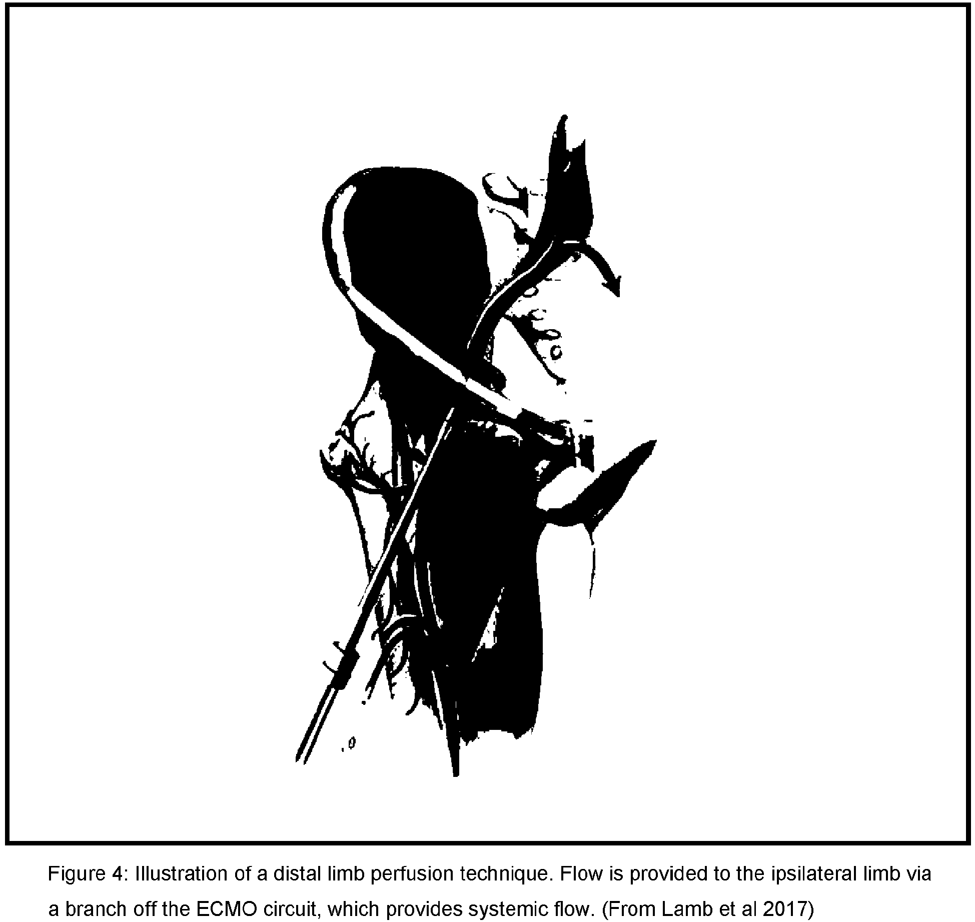 transfusions can result in sensitizing the humoral immune system making it increasingly difficult to find a suitable, compatible donor. Therefore limiting allogeneic blood transfusions is essential.

### Venous and Lymphatic Congestion

The etiology of lymphatic congestion is a complex process, which is not fully understood.

The relationship between arterial, venous, interstitial and lymphatic blood flow is a dynamic complex balance which can be easily influenced by osmotic and oncotic changes as well as blood flow rates. It is important to note that lymph transport, not venous capillary reabsorption, is the main process responsible for interstitial fluid drainage (Mortimer and Levick 2004). Therefore all edema is due to an imbalance between capillary filtration and lymph drainage. It is theorized therefore that in the presence of a femoral venous cannula, reduction in venous drainage from the limb results in subsequent venous engorgement. This then results in increased capillary perfusion pressures with subsequent fluid movement from the venous compartment to the lymphatic vessels. It is thought that the ability of the lymphatic compartment to compensate for this increased interstitial blood flow is low and limb edema ensues. If this is paired with low oncotic pressures (fluid overload is common in patients requiring ECMO as well as Cardiopulmonary bypass for OHS/MICS) increased capillary filtration then overwhelms increased lymph drainage, which subsequently under-compensates.

Interstitial edema in this overwhelmed vascular compartment then increases the oxygen diffusion barrier resulting in cellular ischemia. The inflammatory process then further exacerbates the interstitial edema due to the development of "leaky" capillary beds.

It is believed that this is an under appreciated cause of limb ischemia in peripherally cannulated ECMO and CPB (OHS/MICS) patients and highlights the need to not only maintain arterial blood flow but also venous drainage and therefore prevent overwhelming the lymphatic compartments during the period of cardiopulmonary support. This may also explain why ischemic limb has been seen as a complication in Veno-venous ECMO patients where no arterial cannula is placed.

An object of the inventions is to mitigate the potential for limb ischemia occurring as a consequence to the presence of arterial and venous femoral cannulation. The cannulas are designed to require no additional components other than that of the arterial and venous cannula themselves. The cannulas are designed to be suitable for all femoral cannulation requirements, be it for ECMO, OHS, MICS or other cardiopulmonary systems.

The function of the femoral arterial cannula is to provide retrograde arterial limb flow in the presence of systemic arterial flow via a single cannula.

The function of the femoral venous cannula is to provide retrograde venous limb drainage in the presence of systemic venous flow via a single cannula.

### Femoral Arterial Cannula

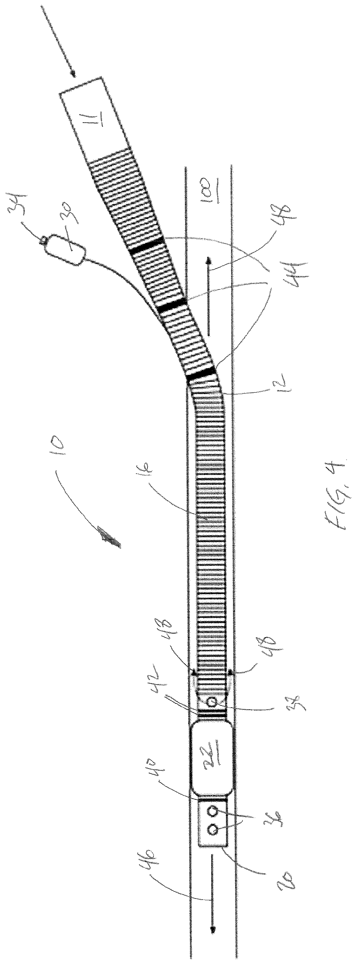

Referring to Figs. 1 to 5 a femoral arterial cannula 10 features a cylindrical plastic body of tubing **12** which preferably has a biocompatible surface coating. The initial length **11** (3/8" OD) remains clear and a 3/8x3/8" luer lock connector is used to connect the initial length with a cardiopulmonary circuit; arterial blood flow enters the cannula **10** through this connector. This connector is a standard perfusion component and is not shown in the figures..

The length of body tubing **12** tapers to one of the varied sizes; e.g. a 15 Fr (5.0mm) body of wire wound tubing **16** or a 17 Fr (5.6mm) body of wire wound tubing or a 19 Fr (6.3mm) body of wire wound tubing 16..

Distal to the length of wire wound tubing **16** is a contiguous length of clear tubing **18,** the tip **20** of which is open ended and is the exit port for systemic arterial flow.

Circumferentially attached to body tubing **12** is a low-profile compliant balloon **22.** Balloon **22** remains parallel to the length of clear tubing **12** when non-inflated. The balloon **22** length is approximately 20mm. The external balloon **22** surface has a drug eluting coating. When inflated the balloon **22** remains cylindrical along its length and is parallel to the body **12** of the cannula **10.** A channel **27,** as best illustrated in Fig. 2, runs the length of body tubing **12** to accommodate and allow a small-bore length of tubing **28** to sit longitudinally flush along body tubing **12.** Small-bore length tubing **28** is attached to balloon **22** at one end and at its other end, to a balloon cuff **30** that includes a two-way valve and female luer port **34.**

Distal to the distal end of the balloon **22** are four circular blood pathway exit ports **36** within contiguous length of clear tubing **18;** as illustrated, two exit ports longitudinally with identical blood flow exit ports at 180 degrees circumferentially. Exit ports **36** are contiguous with the blood pathway for systemic arterial flow.

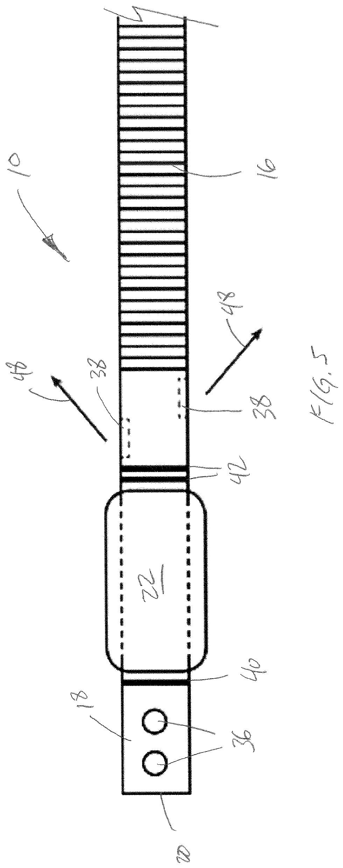

Proximal to the proximal end of the balloon **22** are two exit ports **38** within the contiguous length of clear tubing **18;** exit ports **38** are at 180 degrees circumferentially, and as best illustrated in Fig. 5, are offset longitudinally from each other by 5mm (one exit port is 5mm from the proximal balloon end while the second exit port is 10mm from the proximal balloon end).

Distal to the distal end of the balloon **22** is a circular radio-opaque marker **40** within the body tubing **12.** Proximal to the proximal end of the cannula **10** are two parallel circular radio-opaque markers **42** within the body tubing **12.** Positional markers **44** are identified on the length of the wire wound tubing **16** body of the body tubing **12** to identify cannula positional depth.

The femoral arterial cannula **10** is designed to be inserted either by a surgical cut down or by a percutaneous Seldinger technique. The cannula **10** delivers systemic arterial blood flow **46** along the length of the cannula **10** body through to the cannula tip **20** and the additional blood pathway exit ports **38.** The balloon **22** is designed to seal the femoral artery **100** once inflated. The balloon **22** has a drug eluting coating. The cannula **10** has a biocompatible surface coating. The balloon **22** is de-aired prior to insertion by means of injection and aspiration of saline/contrast solution. Once in situ and inflated the balloon **22** will effectively isolate the blood flow of the proximal exit ports **38** such that they are only available to deliver flow in a retrograde direction down **48** to the ipsilateral limb. With the balloon **22** inflated there is dedicated limb blood flow delivery **48.**

### Femoral Venous Cannula

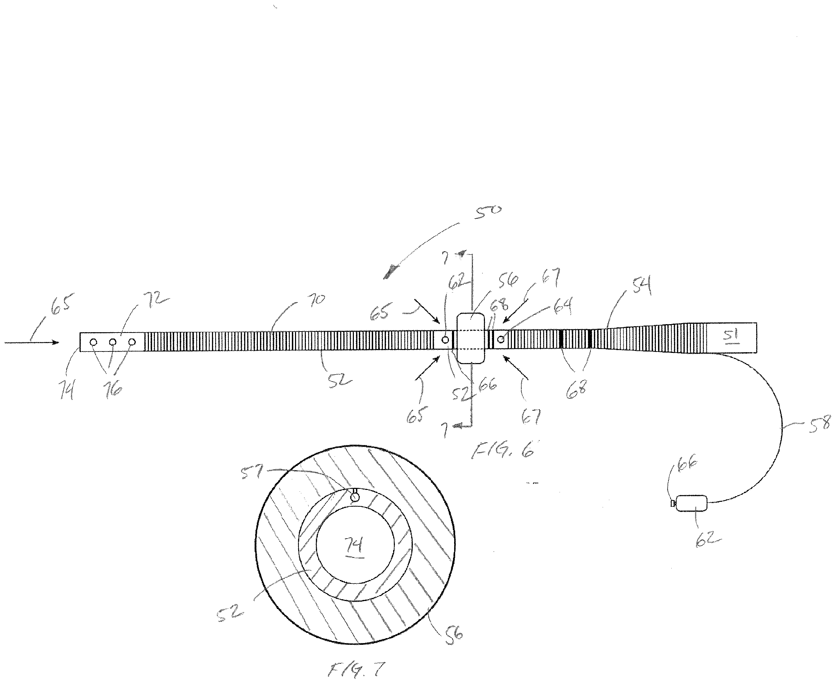

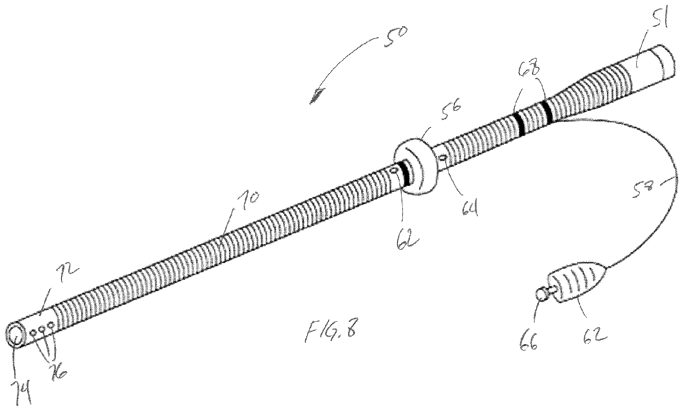

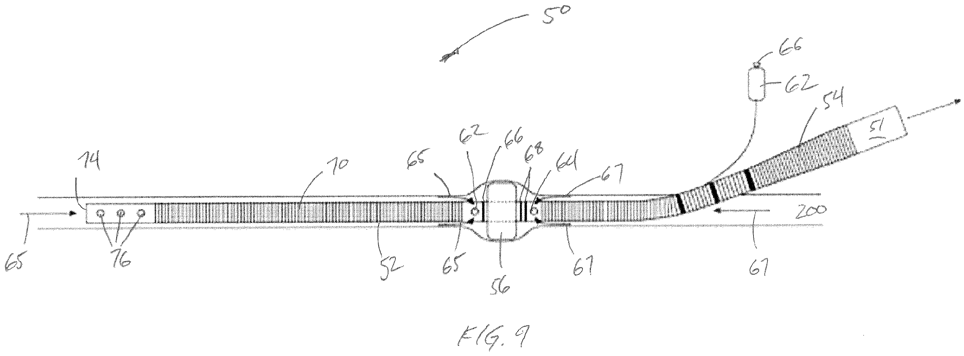

Referring to Figs. 6 to 11, the femoral venous cannula **50** features a cylindrical plastic length of tubing **52** which preferably has a biocompatible surface coating. The initial length **51** (3/8"OD) remains clear with a 3/8×3/8 connector in situ (non luer lock); venous blood flow exits the cannula **50** through this connector which is a standard perfusion item and not shown in the figures. This length of tubing **52** tapers to one of varied sizes: a 23Fr (7.6mm) body of wire wound tubing **54** or a 25Fr (8.3mm) body of wire wound tubing or a 27Fr (9.7mm) body of wire wound tubing..

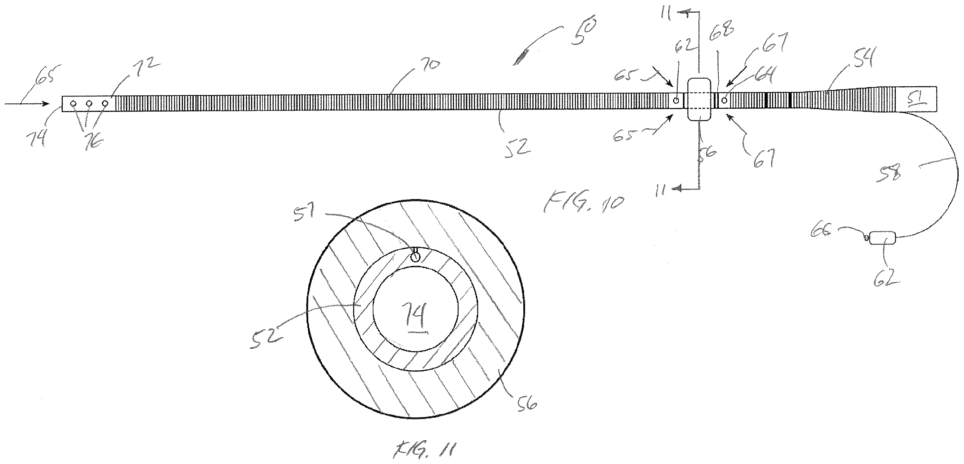

Distal to the length of wire wound tubing **54** is a contiguous length of clear tubing **52.** Attached to clear length of tubing **52** is a broad-profile compliant balloon **56** which remains parallel to the length of clear tubing **52** when non-inflated. The external balloon **56** surface has a drug eluting coating. The balloon **56** length is approximately 12mm. When inflated the balloon **56** remains cylindrical along its length and is parallel to the body tubing **52.** A channel **57,** as best illustrated in Figs. 7 and 11, runs the length of body tubing **52** to accommodate and allow a small-bore length of tubing **58** to sit longitudinally flush along body tubing **52.** Small-bore length tubing **58** is attached to balloon **56** at one end and at its other end, to a balloon cuff **62** that includes a two-way valve and female luer port **66.**

Distal to the distal end of the balloon **56** are two circular blood pathway entry ports **62** within the clear tubing **52;** the two blood entry ports **62** are at 180 degrees circumferentially. These blood entry ports **62** are offset from each other by 5mm (one entry port is 5mm distal from the balloon **56** while the second entry port is 10mm from the balloon **56** end). These entry ports **62** are contiguous with the blood pathway **65** for systemic venous blood flow.

Proximal to the proximal end of the balloon **56** are two blood pathway entry ports **64** within the clear tubing **52,** these ports are at 180 degrees circumferentially but are off-set from each other by 5mm (one entry port is 5mm proximal from the balloon **56** end while the second entry port is 10mm from the balloon end). These ports **64** are circumferentially offset by 90 degrees from the two distal blood entry ports **62.**

Distal to the distal end of the balloon **56** is a single circular radio-opaque marker **66** within the body of the cannula **50.** Proximal to the proximal end of the cannula **10** are two parallel circular radio-opaque markers **68** within the body tubing **52.**

Distal to the clear tubing **52** is a contiguous length of wire wound tubing **70.** Distal to the length of wire wound tubing **70** is a contiguous length of clear tubing **72,** the tip **74** of which is open ended and is the entry port for systemic venous flow **65.** Within this length of clear tubing **72** are six circular blood pathway entry ports **76** within the clear tubing **72,** three entry ports longitudinally with identical exit ports at 180 degrees circumferentially.

Positional markers **68** are identified on the length of the wire wound tubing body **54** of the body tubing **52** to identify cannula positional depth.

The femoral venous cannula **50** is designed to be in varying lengths depending upon its clinical application as well the physical size of the patient. The femoral venous cannula **50** that has application for V-A ECMO, OHS and MICS procedures has three lengths while the model for V-V ECMO has three lengths.

The femoral venous model designed for V-V ECMO is a shorter length than that of the femoral venous cannula designed for V-A ECMO, OHS and MICS. The included schematic has individual design components identical to those of the longer cannula.

The femoral venous cannula **50** is designed to be inserted either by a surgical cut down or by a percutaneous Seldinger technique. The cannula **50** drains systemic venous blood flow **65** along the length of the body tubing **52** from the cannula tip **74** and the additional blood pathway entry ports **62.** The balloon **56** is designed to seal the femoral vein **200** once inflated. The balloon **56** has a drug eluting coating. The cannula **50** has a biocompatible surface coating. The balloon **56** is de-aired prior to insertion by means of injection and aspiration of saline/contrast solution. Once in situ and inflated the balloon **56** will effectively isolate the blood flow of the proximal entry ports **64** such that they are only available to drain venous blood flow **67** from the ipsilateral limb. With the balloon **56** inflated there is dedicated limb flow drainage while the distal entry ports **62** directly above the balloon will drain venous blood from the lower length of the inferior vena cava.

While it is recognized that either cannula can be used individually, the central concept to completely address and prevent limb ischemia is to use both arterial **10** and venous **50** cannula to work synergistically.

### Method of Mitigating Risk of Limb Ischemia

This method does not form part of the claimed invention.

The femoral arterial **10** and venous **50** cannulas have been designed to facilitate the delivery of dedicated arterial limb flow in the presence of dedicated venous limb drainage. A non-perfused limb will suffer the inevitable clinical consequences of ischemia, however a limb that is adequately perfused but is insulted by having inadequate or absent venous drainage will similarly be subjected to a physiological insult that may ultimately be irrecoverable. By facilitating both arterial perfusion along with venous drainage will protect the limb from the adverse consequences of limb ischemia. This identifies and recognizes the multifactorial nature of ischemic limb associated with peripheral cannulation and therefore offers a clinically superior solution opposed to using one cannula in isolation.

While embodiments of the invention have been described and illustrated, such embodiments should be considered illustrative of the invention only. The invention may include variants not described or illustrated herein in detail. Thus, the embodiments described and illustrated herein should not be considered to limit the invention as construed in accordance with the accompanying claims.

### References

Theodosios Bisdas, Gernot Beutel, Gregor Warnecke, Marius M. Hoeper, Christian Kuehn, Axel Haverich, Omke E. Teebken. 2011. Vascular Complications in Patients Undergoing Femoral Cannulation for Extracorporeal Membrane Oxygenation Support. 92:626-31. Ann Thorac Surg.
Jeffrey W. Gander , Jason C. Fisher, Ari R. Reichstein, Erica R. Gross, Gudrun Aspelund, William Middlesworth, Charles J. Stolar. 2010. Limb ischemia after common femoral artery cannulation for venoarterial extracorporeal membrane oxygenation: an unresolved problem. 45, 2136-2140. Journal of Pediatric Surgery.
Yi Juo , Matthew Skancke, Yas Sanaiha, Aditya Mantha, Juan C. Jimenez, Peyman Benharash. 2017. Efficacy of Distal Perfusion Cannulae in Preventing Limb Ischemia During Extracorporeal Membrane Oxygenation: A Systematic Review and Meta-Analysis. 41 (11): E263-E273 Artificial Organs.
Mary Jo Haley, Jason C. Fisher, Alejandro R. Ruiz-Elizalde, Charles J.H. Stolar, Nicholas J. Morrissey, William Middlesworth. 2008. Percutaneous distal perfusion of the lower extremity after femoral cannulation for venoarterial extracorporeal membrane oxygenation in a small child. 44, 437-440. Journal of Pediatric Surgery
Theodore Kalogeris, Christopher P. Baines, Maike Krenz, and Ronald J. Korthuis. 2012. Cell Biology of IschemialReperfusion Injury. 298: 229-317. Int Rev Cell Mol Biol.
Kathleen M. Lamb, Hitoshi Hirose. 2017. Vascular Complications in Extracoporeal Membrane Oxygenation. 33, 813-824. Crit Care Clin.
Kathleen M. Lamb, Paul J. DiMuzio, Adam Johnson, Philip Batista, Neil Moudgill, Megan McCullough, Joshua A. Eisenberg, Hitoshi Hirose, MD, Nicholas C. Cavarocchi. 2016. Arterial protocol including prophylactic distal perfusion catheter decreases limb ischemia complications in patients undergoing extracorporeal membrane oxygenation.65:1074-9. Society for Vascular Surgery. Lukasz A. Adamczyk' Kristiana Gordon' Ivana Kholová' Lorine B. Meijer-Jorna'
Niklas Telinius' Patrick J. Gallagher' Allard C. van der Wal' Ulrik Baandrup. 2016. Lymph vessels: the forgotten second circulation in health and disease. 469:3-17. Virchows Arch.
Hye Ju Yeo, Seong Hoon Yoon, Doosoo Jeon, Yun Seong Kim, Woo Hyun Cho, Dohyung Kim, Seung Eun Lee. 2016. The Utility of Preemptive Distal Perfusion Cannulation During Peripheral Venoarterial Extracorporeal Membrane Oxygenation Support. Vol. 29, No. 4. Journal of interventional cardiology.

## Claims

1. A femoral arterial cannula (10) comprising:
an elongated hollow cannula body (12) having distal (20) and proximal (11) open ends;
a balloon (22) situated on said body adjacent to said distal end;
inflation means connected to said balloon for inflating said balloon;
four blood flow exit ports (36) situated in said cannula body between said balloon and said distal end, two of said distal exit ports in-line with one-another along said cannula body and two other distal exit ports being 180° circumferentially offset from the first two distal exit ports; and
two blood flow exit ports (38) situated in said cannula body between said balloon and said proximal end, said proximal exit ports being 180° circumferentially offset and longitudinally offset from one-another, wherein:
the distal end is a tip of the elongated hollow cannula body, and
the proximal end is at the opposite end of the elongated hollow cannula body from the distal end.

2. The femoral arterial cannula of claim 1 wherein said cannula body is an elongated hollow cylindrical body.

3. The femoral arterial cannula of claim 1 wherein said balloon comprises a low-profile balloon.

4. The femoral arterial cannula of claim 1 wherein said cannula body comprises a taper from said distal to said proximal end.

5. The femoral arterial cannula of claim 1 wherein said cannula body comprises a distal single radio-opaque marker (40), or a proximal double radio-opaque marker (42).

6. The femoral arterial cannula of claim 1 wherein said inflation means comprises:
a small bore tubing (28) connected to said balloon at a first end;
a balloon cuff (30) connected to a second end of said small bore tubing;
a two-way valve connected to said balloon cuff; and
a female luer port (34) connected to said two-way valve.

7. A femoral venous cannula (50) comprising:
an elongated hollow cannula body (52) having distal (74) and proximal (51) open ends;
a balloon (56) situated on said body adjacent to said proximal end;
inflation means connected to said balloon for inflating said balloon;
two blood flow entry ports (62) situated in said cannula body between said balloon and said distal end, said distal entry ports being 180° circumferentially offset and longitudinally offset from one-another along said cannula body; and
two blood flow entry ports (64) situated in said cannula body between said balloon and said proximal end, said proximal entry ports being 180° circumferentially offset and longitudinally offset from one-another and 90° offset from said distal entry ports, wherein:
the distal end is a tip (74) of the elongated hollow cannula body, and
the proximal end is at the opposite end of the elongated hollow cannula body from the distal end.

8. The femoral venous cannula of claim 7 wherein said elongated hollow cannula body is of various lengths.

9. The femoral venous cannula of claim 7 wherein said cannula body is an elongated hollow cylindrical body.

10. The femoral venous cannula of claim 7 wherein said balloon comprises a broad-profile balloon.

11. The femoral venous cannula of claim 7 wherein said cannula body comprises a taper from said distal to said proximal end.

12. The femoral venous cannula of claim 7 wherein said cannula body comprises a distal single radio-opaque marker (66), or a proximal double radio-opaque marker (68).

13. The femoral venous cannula of claim 7 wherein said inflation means comprises:
a small bore tubing (58) connected to said balloon at a first end;
a balloon cuff (62) connected to a second end of said small bore tubing;
a two-way valve connected to said balloon cuff; and
a female luer port (66) connected to said two-way valve.

## Patentansprüche

1. Femorale arterielle Kanüle (10), umfassend:
einen länglichen hohlen Kanülenkörper (12), der ein distales (20) und ein proximales (11) offenes Ende aufweist;
einen Ballon (22), der an dem Körper angrenzend an das distale Ende platziert ist;
ein Aufblähmittel, das mit dem Ballon verbunden ist, zum Aufblähen des Ballons;
vier Blutfluss-Austrittsöffnungen (36), die in dem Kanülenkörper zwischen dem Ballon und dem distalen Ende platziert sind, wobei zwei der distalen Austrittsöffnungen in Reihe miteinander entlang des Kanülenkörpers sind und zwei andere distale Austrittsöffnungen um 180° umlaufend versetzt von den ersten beiden distalen Austrittsöffnungen sind; und
zwei Blutfluss-Austrittsöffnungen (38), die in dem Kanülenkörper zwischen dem Ballon und dem proximalen Ende platziert sind, wobei die proximalen Austrittsöffnungen um 180° umlaufend versetzt und in Längsrichtung voneinander versetzt sind, wobei:
das distale Ende eine Spitze des länglichen hohlen Kanülenkörpers ist, und
das proximale Ende am gegenüberliegenden Ende des länglichen hohlen Kanülenkörpers vom distalen Ende ist.

2. Femorale arterielle Kanüle nach Anspruch 1, wobei der Kanülenkörper ein länglicher hohler zylindrischer Körper ist.

3. Femorale arterielle Kanüle nach Anspruch 1, wobei der Ballon einen Ballon mit niedrigem Profil umfasst.

4. Femorale arterielle Kanüle nach Anspruch 1, wobei der Kanülenkörper einen Kegel vom distalen zum proximalen Ende umfasst.

5. Femorale arterielle Kanüle nach Anspruch 1, wobei der Kanülenkörper einen distalen einzelnen radio-opaken Marker (40) oder einen proximalen doppelten radio-opaken Marker (42) umfasst.

6. Femorale arterielle Kanüle nach Anspruch 1, wobei das Aufblähmittel umfasst:
eine Leitung (28) mit kleinem Durchmesser, die mit dem Ballon an einem ersten Ende verbunden ist;
eine Ballonmanschette (30), die mit einem zweiten Ende der Leitung mit kleinem Durchmesser verbunden ist;
ein Zweiwegeventil, das mit der Ballonmanschette verbunden ist; und
eine weibliche Luer-Öffnung (34), die mit dem Zweiwegeventil verbunden ist.

7. Femorale venöse Kanüle (50), umfassend:
einen länglichen hohlen Kanülenkörper (52), der ein distales (74) und ein proximales (51) offenes Ende aufweist;
einen Ballon (56), der an dem Körper angrenzend an das proximale Ende platziert ist;
ein Aufblähmittel, das mit dem Ballon verbunden ist, zum Aufblähen des Ballons;
zwei Blutfluss-Eintrittsöffnungen (62), die in dem Kanülenkörper zwischen dem Ballon und dem distalen Ende platziert sind, wobei die distalen Eintrittsöffnungen um 180° umlaufend versetzt und in Längsrichtung voneinander entlang des Kanülenkörpers versetzt sind; und
zwei Blutfluss-Eintrittsöffnungen (64), die in dem Kanülenkörper zwischen dem Ballon und dem proximalen Ende platziert sind, wobei die proximalen Eintrittsöffnungen um 180° umlaufend versetzt und in Längsrichtung voneinander versetzt und um 90° von den distalen Eintrittsöffnungen versetzt sind, wobei:
das distale Ende eine Spitze (74) des länglichen hohlen Kanülenkörpers ist, und
das proximale Ende am gegenüberliegenden Ende des länglichen hohlen Kanülenkörpers vom distalen Ende ist.

8. Femorale venöse Kanüle nach Anspruch 7, wobei der längliche hohle Kanülenkörper unterschiedliche Längen aufweist.

9. Femorale venöse Kanüle nach Anspruch 7, wobei der Kanülenkörper ein länglicher hohler zylindrischer Körper ist.

10. Femorale venöse Kanüle nach Anspruch 7, wobei der Ballon einen Ballon mit breitem Profil umfasst.

11. Femorale venöse Kanüle nach Anspruch 7, wobei der Kanülenkörper einen Kegel vom distalen zum proximalen Ende umfasst.

12. Femorale venöse Kanüle nach Anspruch 7, wobei der Kanülenkörper einen distalen einzelnen radio-opaken Marker (66) oder einen proximalen doppelten radio-opaken Marker (68) umfasst.

13. Femorale venöse Kanüle nach Anspruch 7, wobei das Aufblähmittel umfasst:
eine Leitung (58) mit kleinem Durchmesser, die mit dem Ballon an einem ersten Ende verbunden ist;
eine Ballonmanschette (62), die mit einem zweiten Ende der Leitung mit kleinem Durchmesser verbunden ist;
ein Zweiwegeventil, das mit der Ballonmanschette verbunden ist; und
eine weibliche Luer-Öffnung (66), die mit dem Zweiwegeventil verbunden ist.

## Revendications

1. Canule artérielle fémorale (10) comprenant :
un corps de canule creux allongé (12) ayant des extrémités ouvertes distale (20) et proximale (11) ;
un ballonnet (22) situé sur ledit corps adjacent à ladite extrémité distale ;
un moyen de gonflage relié audit ballonnet pour gonfler ledit ballonnet ;
quatre orifices de sortie de flux sanguin (36) situés dans ledit corps de canule entre ledit ballonnet et ladite extrémité distale, deux desdits orifices de sortie distaux étant alignés l'un avec l'autre le long dudit corps de canule et deux autres orifices de sortie distaux étant décalés de manière circonférentielle de 180° par rapport aux deux premiers orifices de sortie distaux ; et
deux orifices de sortie de flux sanguin (38) situés dans ledit corps de canule entre ledit ballonnet et ladite extrémité proximale, lesdits orifices de sortie proximaux étant décalés de 180° de manière circonférentielle et longitudinale l'un par rapport à l'autre,
dans laquelle :
l'extrémité distale est une pointe du corps de canule creux allongé, et
l'extrémité proximale se trouve au niveau de l'extrémité opposée du corps de canule creux allongé de l'extrémité distale.

2. Canule artérielle fémorale de la revendication 1, dans laquelle ledit corps de canule est un corps cylindrique creux allongé.

3. Canule artérielle fémorale de la revendication 1, dans laquelle ledit ballonnet comprend un ballonnet à profil bas.

4. Canule artérielle fémorale de la revendication 1, dans laquelle ledit corps de canule comprend un rétrécissement de ladite extrémité distale à ladite extrémité proximale.

5. Canule artérielle fémorale de la revendication 1, dans laquelle ledit corps de canule comprend un marqueur radio-opaque unique distal (40) ou un marqueur radio-opaque double proximal (42).

6. Canule artérielle fémorale de la revendication 1, dans laquelle ledit moyen de gonflage comprend :
un tube de petit calibre (28) relié audit ballonnet au niveau d'une première extrémité ;
une manchette de ballonnet (30) reliée à une seconde extrémité dudit tube de petit calibre ;
une soupape à deux voies reliée à ladite manchette de ballonnet ; et
un orifice Luer femelle (34) relié à ladite soupape à deux voies.

7. Canule veineuse fémorale (50), comprenant :
un corps de canule creux allongé (52) ayant des extrémités ouvertes distale (74) et proximale (51) ;
un ballonnet (56) situé sur ledit corps adjacent à ladite extrémité proximale ;
un moyen de gonflage relié audit ballonnet pour gonfler ledit ballonnet ;
deux orifices d'entrée de flux sanguin (62) situés dans ledit corps de canule entre ledit ballonnet et ladite extrémité distale, lesdits orifices d'entrée distaux étant décalés de 180° de manière circonférentielle et longitudinale l'un par rapport à l'autre le long dudit corps de canule ; et
deux orifices d'entrée de flux sanguin (64) situés dans ledit corps de canule entre ledit ballonnet et ladite extrémité proximale, lesdits orifices d'entrée proximaux étant décalés de 180° de manière circonférentielle et longitudinale l'un par rapport à l'autre et décalés de 90° par rapport auxdits orifices d'entrée distaux,
dans laquelle :
l'extrémité distale est une pointe (74) du corps de canule creux allongé, et
l'extrémité proximale se trouve au niveau de l'extrémité opposée du corps de canule creux allongé de l'extrémité distale.

8. Canule veineuse fémorale de la revendication 7, dans laquelle ledit corps de canule creux allongé est de différentes longueurs.

9. Canule veineuse fémorale de la revendication 7, dans laquelle ledit corps de canule est un corps cylindrique creux allongé.

10. Canule veineuse fémorale de la revendication 7, dans laquelle ledit ballonnet comprend un ballonnet à profil large.

11. Canule veineuse fémorale de la revendication 7, dans laquelle ledit corps de canule comprend un rétrécissement de ladite extrémité distale à ladite extrémité proximale.

12. Canule veineuse fémorale de la revendication 7, dans laquelle ledit corps de canule comprend un marqueur radio-opaque unique distal (66) ou un marqueur radio-opaque double proximal (68).

13. Canule veineuse fémorale de la revendication 7, dans laquelle ledit moyen de gonflage comprend :
un tube de petit calibre (58) relié audit ballonnet au niveau d'une première extrémité ;
une manchette de ballonnet (62) reliée à une seconde extrémité dudit tube de petit calibre ;
une soupape à deux voies reliée à ladite manchette de ballonnet ; et
un orifice Luer femelle (66) relié à ladite soupape à deux voies.
